# EUROPÄISCHE PATENTSCHRIFT

(11) **EP 1 920 055 B1**
(45) Veröffentlichungstag und Bekanntmachung des Hinweises auf die Patenterteilung: **30.04.2014**
(21) Anmeldenummer: 06777971.0
(22) Anmeldetag: 25.07.2006
(51) Int. Cl.: C12Q 1/68

(54) **VERFAHREN ZUR GEWINNUNG VON NUKLEINSÄUREN AUS BLUT**
METHOD FOR EXTRACTING NUCLEIC ACID FROM BLOOD
PROCEDE POUR ISOLER DES ACIDES NUCLEIQUES DANS LE SANG

(30) Priorität: 24.08.2005 DE 102005040259
(43) Veröffentlichungstag der Anmeldung: 14.05.2008
(73) Patentinhaber: Qiagen GmbH, 40724 Hilden (DE)
(72) Erfinder: ERBACHER, Christoph, 42781 Haan (DE); HIMMELREICH, Ralf, 40764 Langenfeld (DE); PEIST, Ralf, 40229 Düsseldorf (DE); EVANS HAALAND, Ingerlise, N-2007 Kjeller (NO); HARDERSEN, Hege, N-1361 Osteras (NO)
(86) Internationale Anmeldenummer: PCT/EP2006/064652
(87) Internationale Veröffentlichungsnummer: WO 2007/023057

(56) Entgegenhaltungen:
- WO-A-01/30995
- WO-A-97/11160
- WO-A-99/07749
- WO-A-2005/066361
- US-A- 5 508 164
- FOTH, HANS-JOCHEN: 'Auszüge aus dem Chemie-Lexikon Roempp (Onlineversion) zur "Adsorption" bzw. "Adhäsion".', [Online] Roempp Online, Version 3.13 Gefunden im Internet: <URL:www.roempp.com/prod/roempp.php>

## Beschreibung

Die vorliegende Erfindung betrifft ein verbessertes Verfahren zur Isolierung von Nukleinsäuren insbesondere genomischer Desoxyribonukleinsäure (DNA) aus Blut.

Aus dem Stand der Technik sind Verfahren zur Reinigung und Isolierung von Nukleinsäuren insbesondere genomischer DNA aus sog. Vollblut bekannt. Diese erweisen sich jedoch als sehr komplex in ihrer Anwendungsweise und sind darüber hinaus sehr arbeitsintensiv und zeitaufwendig. Daneben erfordern die meisten Methoden neben einer Isolierung des Zellmaterials oder der Zellkerne einen relativ langen Inkubationszeitraum in Gegenwart von Proteinasen, gefolgt von - ggf. mehreren - Phenolextraktionen (Molecular Cloning, A Laboratory Manual, Maniatis et al., pg. 9,17-9,18 sowie Nucl. Acid Res. 3, 2303 - 2306).

Angesichts dieser Nachteile wurden in der Folgezeit alternative Methoden entwickelt, mit denen nicht nur der Aufwand minimiert werden sollte, sondern auch die Ausbeute an Nukleinsäure bzw. genomischer DNA sowie deren Reinheitsgrad erhöht werden sollte. Derartige Methoden basieren auf dem Einsatz von sog. chaotropen Salzen (Nucl. Acid Res. 15, 9611, 1987 sowie Anal. Biochem. 180, 276 - 278, 1989).

So wird u.a. in der Europäischen Offenlegungsschrift EP 0 389 063 ein Verfahren offenbart, in dem die zu untersuchende Blutprobe mit einer chaotropen Substanz in Gegenwart eines - in Form von Partikeln vorliegenden - Matrixmaterials, wie zum Beispiel Silica, behandelt wird. Dabei wird die Erkenntnis eingesetzt, dass die Nukleinsäuren unter diesen Reaktionsbedingungen aus den Zellen freigesetzt werden und dann an geeignete - auf Silica basierende Matrixmaterialien - binden. Danach kann das die Nukleinsäuren aufweisende Matrixmaterial - z.B. auf dem Wege der Zentrifugation - aus der Reaktionslösung abgetrennt werden. Nach dem Entfernen des Überstandes wird das Matrixmaterial einem oder erforderlichenfalls mehreren Waschschritten unterworfen.

Die oben beschriebenen Methoden bergen allerdings diejenigen Nachteile in sich, dass sie sehr zeitaufwendig sind und unerwünschte Zentrifugationsschritte beinhalten, die zwangsläufig mit einem Materialtransfer verbunden sind, welcher wiederum für jede einzelne Probe aufwendig und daneben mit einer Kontaminationsgefahr verbunden ist. Daneben weist diese Verfahrensweise den schwerwiegenden Nachteil auf, dass sie nicht automatisierbar ist.

Die Aufgabe der vorliegenden Erfindung besteht somit darin, die Nachteile der aus dem Stand der Technik bekannten Verfahren zu vermeiden und ein Verfahren zur Isolierung von Nukleinsäuren bevorzugt von genomischer DNA aus Blut zur Verfügung zu stellen, welches insbesondere zeitaufwendige und vor allem arbeitsaufwendige Zentrifugationsschritte vermeidet und welches insbesondere automatisierbar ist. Danbeben soll die Kontaminationsgefahr und der anfallende biologisch kontaminierte Abfall und auf ein Minimum verringert werden. Des Weiteren sollen die so gewonnenen Nukleinsäuren bzw. genomische DNA in einer Form verfügbar sein, die sie für Folgereaktionen - wie z.B. Amplifikationsreaktionen insbesondere PCR - ohne zusätzliche Verfahrensschritte verfügbar macht. Diese Forderung erwächst aus dem Bedarf an genomischer DNA für zahlreiche verschiedene Untersuchungsmethoden wie z.B. Genom-Typisierungen (Genotyping) etc.

Die Anforderung, die an die vorgelagerte Isolierung der genomischen DNA gestellt wird, besteht insbesondere darin, dass die genomische DNA, die u.a. das essentielle Ausgangsmaterial der Genom-Typisierung darstellt, möglichst schnell aufgereinigt wird und ohne Zeitverzögerung und ohne mit fremder Nukleinsäure kontaminiert zu werden in der gewünschten Folgereaktion eingesetzt werden kann - insbesondere:
PCR basierte Genomtypisierung;
SNP-Analysen im humanen Genom;
Erstellung eines genetischen Fingerabdrucks (Forensik, Kriminaltechnik);
Diagnostische Genotypisierung (z.B. zystische Fibrose; Faktor V Leiden);
Durchführung von vielfacher oder Multiplex-PCR aus einer Probe zum Nachweis von Markern;
Restriktionslängenpolymorphismen basierend auf primärer PCR Amplifikation;
Durchführung von quantitativen PCRs.

Gelöst werden die vorbeschriebenen Aufgaben durch das in der vorliegenden Erfindung vorgeschlagene Verfahren, bei dem die - zu analysierende - Blutprobe zunächst mit einem Erythrozyten-Lysepuffer in einem Reaktionsgefäß in Kontakt gebracht wird, welches mit einer Beschichtung versehen ist, die durch die Lyse freigesetzten Zellkerne und Mitochondrien adsorbieren kann.

Daneben kann das adsorbierende Material auch in Form eines nicht an den Begrenzungswänden des Reaktionsgefäßes fixierten Matrixmaterials in dem zugegen sein. Vorteilhafterweise ist das innen beschichtete Reaktionsgefäß in der Weise ausgestaltet, dass es die Durchführung einer Amplifikationsreaktion bzw. PCR ermöglicht, und - in einer besonderen Ausführungsform - eine automatisierte Durchführung der gewünschten Reaktion von mehreren bzw. von einer Vielzahl von Proben ermöglicht.

Überraschenderweise wurde gefunden, dass weiße Blutzellen und sowie durch die Erythrozytenlyse freigesetzte Zellbestandteile - wie Zellkerne und Mitochondrien - in Gegenwart von Erythrozyten-Lysepuffern an polyanionische Strukturen aufweisende Polymere, insbesondere anionische Polymere, die funktionelle Gruppen von Carbonsäuren oder anderen Säuren - wie Sulfon- oder Phosphonsäuren oder Phenole als Säuren im weitesten Sinne - aufweisen, binden. Dabei lysieren die erfindungsgemäß eingesetzten Erythrozyten-Lysepuffer vornehmlich Erythrozyten, wobei weiße Blutkörperchen im wesentlichen intakt bleiben, bzw. letztere werden nur so insoweit lysiert, dass sie als sog. Zellorganellen (Zellkerne und Mitochondrien) verbleiben. Die Zellorganellen müssen dabei nicht vollständig intakt bleiben, jedoch zumindest soweit, dass eine Adsorption der Oberfläche der Zellorganellen an die eine polyanionische Struktur aufweisenden Polymere erfolgen kann. Die Zerstörung der Zellorganellen sollte dabei auch noch nicht so weit fortgeschritten sein, dass die Nukleinsäure aus den Zellorganellen freigesetzt wurde, da sich gezeigt hat, dass die Nukleinsäure als solche schlecht an die genannten Polymere bindet. Auch Zelldebris, in die DNA eingeschlossen ist, kann an die Polymermatrix gebunden werden. Alle anderen störenden Blutbestandteile - wie z.B. Hämoglobin - können nach der Lyse durch Entfernen der Reaktionslösung bzw. durch Waschen der polyanionischen Polymermatrix mit geeigneten Waschpuffern weitestgehend entfernt werden.

Die Adsorption der oben beschriebenen Zellorganellen an die Matrix anstelle einer Bindung der Nukleinsäuren direkt, wie es verschiedentlich im Stand der Technik beschrieben ist, und die Verwendung von polyanionische Strukturen aufweisenden Polymeren zu diesem Zweck hat den Vorteil, dass der pH-Wert im Lyseschritt im Vergleich zum pH-Wert bei einer optional anschließend erfolgenden PCR-Reaktion nicht geändert werden muss, sondern gleich bleiben kann. Dadurch kann es vermieden werden, zusätzliche Verfahrensschritte einzuführen, die der Neutralisation oder der Einstellung eines anderen pH-Wertes dienen.

Um die Blutprobe zu stabilisieren und etwaige Zersetzungsreaktionen zu verhindern oder zu verzögern kann es vorteilhaft sein, die Blutprobe möglichst kurz nach der Entnahme einzufrieren und die gefrorene Probe bzw. die aufgetaute Probe dem erfindungsgemäßen Verfahren zu unterziehen.

Die für die Lyse erforderlichen Lysepuffer sind aus dem Stand der Technik bekannt und daneben auch kommerziell erhältlich - z.B. "QIAGEN Buffer FG1", "QIAGEN Buffer C1" oder Gentra RBC-Lyselösung. Prinzipiell ist allerdings die Verwendung eines jeden Puffers möglich, dessen Bestandteile in der Lage sind, Erythrozyten zu lysieren. Als Beispiel sei eine Pufferlösung enthaltend 155 mM Ammoniumchlorid und 10 mM Kaliumhydrogencarbonat genannt. Weitere geeignete Puffer sind dem Fachmann bekannt und können Salze anorganischer oder organischer Säuren enthalten. Sie basieren dabei vorzugsweise auf wässerigen Lösungen von Alkali-Erdalkalimetallhalogeniden - wie z.B. Kalium- und Magnesiumchlorid, ergänzt um einen pH-Puffer, wie z.B. Tris, ein Detergenz und ggf. einen Komplexbildner. Besonders bevorzugte Salze anorganischer oder organischer Säuren sind daneben sind Ammoniumsalze wie z.B. Ammoniumchlorid, Ammoniumsulfat oder organische Ammoniumsalze wie Ammoniumtartrat in Konzentrationen zwischen 100 und 500 mM, sowie anionische, neutrale oder kationische Detergenzien in Konzentrationen von 0,01 Gew-% - 5 Gew-%. Bevorzugte Detergenzien sind beispielsweise SDS, Chaps, Tween 20, Triton oder Catrimox.

Als Beschichtungsmaterialien sind prinzipiell alle anionische Strukturen ausbildenden Polymere geeignet, wobei carboxylierte Polymere, Co- oder Terpolymere oder Mischungen dieser Polymere bevorzugt werden. Beispielsweise können Copolymere auf der Basis Styrol, Vinylmethylether, unverzweigten oder verzweigten Alkenen - wie z.B. 1-Octadecen oder Isopropylen und Maleinsäure oder Acrylsäure, wobei die Carboxylfunktionen ggf. in unterschiedlichen Graden verestert sein können, zum Einsatz kommen. Bevorzugt seien als Beispiele folgende Acrylsäurealkylester genannt: Methylacrylat, Ethylacrylat, Vinylacrylat, Propylacrylat, Butylacrylat, Hexylacrylat, Octylacrylat, Decylacrylat, Dodecylacrylat, Myristylacrylat, Laurylacrylat, Cetylacrylat, Stearylacrylat, Methylmethacrylat, Ethylmethacrylat, Propylmethacrylat, Butylmethacrylat, Isobutylmethacrylat, Hexylmethacrylat, 2-Ethyl-hexylacrylat, 2-Ethyl-hexylmethacrylat Phenylemethacrylat, Octylmethacrylat, Decylmethacrylat, Dodecylmethacrylat, Myristylmethacrylat, Laurylmethacrylat, Cetylmethacrylat, worunter der Acrylsäuremethylester besonders bevorzugt wird.

Bevorzugt sind die verzweigten oder unverzweigten C₁- bis C₁₂-Alkylester der Acryl- oder Methacrylsäure, insbesondere Methylmethacrylat, Ethylmethacrylat, Butylmethacrylat, 2-Ethylhexylmethacrylat, Methylacrylat, Ethylacrylat, Butylacrylat und 2-Ethylhexyacrylat oder Maleinsäurealkylester - wie z.B.: Maleinsäuremethylester.

Daneben ist es möglich, andere Polylanionenstrukturen ausbildende Polymere einzusetzen, die beispielsweise Sulfon- oder Phosphonsäuregruppen oder Phenolreste oder Mischungen derselben aufweisen; stellvertretend sei als Beispiel Polystyrolsulfonsäure in Form des Homopolymers oder als Bestandteil eines Copolymers genannt.

Obwohl die Phenolverbindungen auch Bestandteil der Hauptkette sein können, ist es bevorzugt, dass sich diese an Seitenketten des Polymerrückgrates befinden. Generell ist es möglich, die Phenolgruppe auch nach der Polymerisierung in das Polymer einzuführen. Eine besonders homogene Verteilung wird jedoch erzielt, wenn die Phenolgruppen bereits Bestandteil des Monomers sind. So können sie beispielsweise über Substituenten wie Aminreste an polymerisierbare Monomere wie Acrylsäure, Methacrylsäure oder deren Derivate gebunden werden. Um einen größeren Abstand des Phenols in der Seitenkette zum Polymerrückgrat zu erhalten können weitere Verbindungen zwischen dem polymerisierbaren Bestandteil und dem Phenolrest als Brücke fungieren. Bei einer besonders bevorzugt verwendeten Phenolverbindung handelt es sich um Tyramin.

Weitere geeignete Polymere stellen Polyester dar, die zur Ausbildung einer polyanionischen Struktur in der Lage sind. Generell können die Monomere an sich die Gruppen aufweisen, die nach der Polymerisation anionische Stellen im Polymer darstellen oder ausbilden. Daneben können die Polyester auch nachträglich noch mit Anionen ausbildenden Gruppen versehen werden, z.B. über ungesättigte Stellen oder andere funktionelle Gruppen, die im Polyester vorliegen.

Vorzugsweise werden als Monomere Dicarbonsäuren wie Bernsteinsäure, Adipinsäure, Phthalsäure oder Maleinsäure und als Alkohol Weinsäure oder andere Di- oder Trialkohole, die mindestens eine weitere anionsche Funktionalität enthalten, verwendet. Bei Verwendung von Maleinsäure als Monomer können auch Di- oder Trialkohole ohne weitere anionischen Funktionalitäten zur Polymerisation verwendet werden, wenn die im Polymer verbleibende Doppelbindung von der Maleinsäure beispielsweise genutzt wird, um nachträglich Anionen ausbildende Gruppen einzuführen, wie beispielsweise Acrylsäure oder Methacrylsäure.

Das zur Ausbildung einer polyanionischen Struktur befähigte Copolymer wird vorzugsweise durch ein carboxyliertes Polymer auf der Basis von Styrol und Maleinsäureanhydrid verkörpert. Dabei weist das Copolymer Copolymer 5 bis 95 Gew.-%, bevorzugt 25 bis 95 Gew.-% und besonders bevorzugt 50 bis 95 Gew.-% an Maleinsäureeinheiten auf.

Das zur Ausbildung dass das zur Ausbildung einer polyanionischen Struktur befähigte Copolymer wird daneben ebenfalls vorzugsweise durchein carboxyliertes Polymer auf der Basis von Methylvinylether und Maleinsäure verkörpert. Das Copolymer kann dabei zum Beispiel als

### Poly(methylvinylether-alt-maleinsäure)

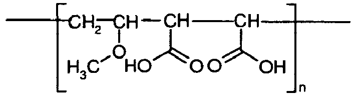

vorliegen, deren Carboxylgruppen teilweise - z.B. mit einer Methylgruppe - verestert sein können.

Dabei können Poly(methylvinylether-alt-maleinsäure)-Copolymere mit einem Molekulargewicht in einem Bereich von 1.0·10³ bis 2.5·10⁶, bevorzugt 1.0·10⁴ bis 2.2·10⁶ und besonders bevorzugt in einem Bereich von 1.9·10⁶ bis 2.1·10⁶ liegt.

Namentlich werden besonders bevorzugt:
Poly(methyl vinyl ether-alt-maleinsäure) MW: 1.980.000
Poly(isobutylen-alt-maleinsäure) MW: 3.250.000
Polystyrol-co-maleinsäure, 50 Gewichtsprozent Maleinsäure
Polystyrol-co-maleinsäure, 25 Gewichtsprozent Maleinsäure
Polystyrol-co-maleinsäure, 14 Gewichtsprozent Maleinsäure
Polyisopren-graft-maleinsäure 7 Gewichtsprozent Maleinsäure
Poly(maleinsäure-1-octadec-1-en) im Molverhältnis 1:1
Poly(methylvinylether-alt maleinsäure) MW: 1.250.000
Polystyrol-alt-maleinsäure teilweise als Methylester
Poly(acrylsäure-co-acrylsäuremethylester)
Polystyrolsulfonsäure

Die Herstellung carboxylierter Polymere ist aus dem Stand der Technik wohlbekannt - da diese Polymere in einer Vielzahl anderer technischer Anwendungen eingesetzt werden, sind sie zu einem großen Teil auch kommerziell erhältlich.

Weitere Polymere können über radiaklische Polymerisation mit Carboxylgruppenaufweisenden Monomeren hergestellt werden.

Die polyanionischen bzw. carboxylierten Polymere können auf vielfältige Art und Weise auf die Begrenzungswand/-wände des Reaktionsgefäßes aufgebracht werden, in flüssigem Zustand bzw. in Form einer Suspension in dem aus der Lyse resultierenden Gemisch zugegen sein oder in Form eines sog. Dippsticks vorliegen. So kann die Beschichtung mit einer Polymerschmelze zu der gewünschten funktionalisierten Schicht führen. Daneben besteht die Möglichkeit - sofern das Reaktionsgefäß mittels Spritzguss hergestellt wird - das Polymer als Additiv vor dem Spritzguss der für den Spritzguss vorgesehenen Masse zuzufügen; ebenso besteht die Möglichkeit, alle oder bestimmte Teile der Spritzgussform mit dem Polymer vor dem Spritzguss zu beschichten, wobei nach dem Ausbilden des Reaktionsgefäßes die gewünschten Beschichtungen erhalten werden. Daneben besteht die Möglichkeit, eine Folie aus dem die polyanionischen Strukturen ausbildenden Polymer an der Innenwand des Reaktionsgefäßes anzubringen und z.B. durch sog. Triefzugverfahren in die gewünschte geometrische Form zu bringen. Bevorzugt wird daneben die Beschichtung mit dem polyanionischen Polymer, das in einem geeigneten Lösungsmittel gelöst ist. Des Weiteren besteht die Möglichkeit, dass das Polymer in der Lösung zunächst als in Form eines sog. Precursors vorliegt, aus dem im Zuge des Reaktionsverlaufs erst das die gewünschte Struktur aufweisende Polymer erst gebildet wird.

Des weiteren besteht die Möglichkeit - bei Auswahl dafür geeigneter Polymere, die dem Fachmann bekannt sind - nach der Herstellung des zunächst nicht funktionalisierten Reaktionsgefäßes -, die gewünschte Funktionalisierung der Oberfläche mit aus dem Stand der Technik bekannten "nasschemischen Verfahren" (Schwefelsäure/Wasserstoffperoxid) oder auf dem Wege Atmosphärendruck-Plasmabehandlung zu erzielen.

Im nachfolgenden Schritt kann sich beispielsweise eine wie auch immer geartete Amplifikationsreaktion.- beispielsweise eine PCR - anschließen, die unter den aus dem Stand der Technik wohlbekannten Reaktionsbedingungen durchgeführt werden kann. Hierzu sind keine besonderen Verfahrungsanpassungen erforderlich. Beim ersten Erhitzen der Reaktionslösung auf eine Temperatur in einem Intervall von 45 bis maximal 100 °C, bevorzugt auf eine Temperatur in einem Intervall von 80 bis 98 °C und besonders bevorzugt auf 94 - 96 °C werden die Nukleinsäuren bzw. die genomische DNA (thermische Lyse) freigesetzt und steht somit in der nachfolgenden PCR zur Verfügung.

In einer weiteren Ausführungsform kann auf eine Beschichtung oder Derivatisierung oder Gefäßoberfläche verzichtet werden, wenn das zur Ausbildung von polyanionischen Strukturen befähigte Polymer als zusätzliche Komponente dem Lysepuffer oder dem zu lysierenden Gemisch zugefügt wird. Die Bindung von gDNA-haltigen Zellbestandteilen wie, Mitochondrien und Zellkernen findet dann nicht an der PCR-Gefäßwandung statt, sondern direkt in der flüssigen Phase. Dies führt zu Komplexen bestehend aus den genannten Zellbestandteilen und dem carboxylierten Polymer die während der Lyse und Inkubation im PCR-Gefäß an dessen Wandung adhärieren können. Die Beschichtung findet also simultan während der Lyse statt d.h. bereits in der flüssigen Phase gebundene Zellbestandteile werden zusammen mit dem carboxylierten Polymer an der Gefäßwand abgeschieden.

In allen Ausführungsformen bleiben nach der Lyse und ggf. dem Waschen der polyanionischen Polymere die gDNA-haltigen Zellbestandteile zurück und können - wie beschrieben - weiter behandelt bzw. detektiert und quantifiziert werden.

Somit ermöglicht das erfindungsgemäße Verfahren mit der Durchführung der Analyse von der Lyse der Blutprobe bis zum Beginn der gewünschten Folgereaktion in einem Gefäß nicht nur eine deutliche Reduktion der Anzahl der Arbeitsschritte und der Kontaminationsgefahr der Proben, sondern auch damit einhergehend eine deutliche Verminderung an biologisch kontaminierten Abfall, da die Proben nicht mehr umpipettiert werden müssen.

Eine weitere Materialersparnis ergibt sich aus dem Umstand, dass die aktuell am Markt verfügbaren Kit-Formate für die Isolation von relativ großen Mengen gDNA ausgelegt sind (typischerweise 20 µg). Dies ist für viele Einsatzgebiete (z.B. Analyse einzelner Marker) überdimensioniert. Die erfindungsgemäße Lösung kann für die Präparation von 10 ng gDNA ausgelegt werden und spart so zusätzlich Material ein. Durch beide Einsparungseffekte (weniger Arbeitsschritte, kleinere Kits) vermindert sich insgesamt der Materialeinsatz an Spezial-Plastikartikeln (z.B. an gestanzten Silicamembranen im Verbund mit Polypropylen Spritzgußteilen) und Pufferlösungen erheblich. Zum Zweiten sinkt durch den verringerten Materialeinsatz auch der Energieaufwand für die Herstellung der entsprechenden Vorprodukte.

Obwohl es aus Gründen der Arbeitserleichterung und der Zeitersparnis bzw. der Möglichkeit zur Automatisierung bevorzugt ist, jegliche Zentrifugationsschritte wegzulassen besteht jedoch auch bei dem erfindungsgemäßen Verfahren die Möglichkeit, zusätzlich ein oder mehrere Zentrifugationsschritte zu integrieren. Vorteilhafterweise finden diese nach dem in Kontakt Bringen der Blutprobe mit dem Erythrozyten-Lysepuffer und vor der Entfernung der aus der Lyse resultierenden Reaktionsmischung statt. Es hat sich gezeigt, dass durch die Zentrifugation die Zellorganellen deutlich fester an die Matrix gebunden werden als ohne einen entsprechenden Zentrifugationsschritt. Dies hat den Vorteil, dass bei dem Waschschritt der Verlust an Zellorganellen, die sich beim Waschen von der Matrix lösen und zusammen mit der Waschlösung entfernt werden, und damit auch der Verlust an isolierter Nukleinsäure, verringert wird.

Bei einer Integration eines Zentrifugationsschrittes in das erfindungsgemäße Verfahren werden nicht unerwünschte Bestandteile der Lyselösung sedimentiert und die gewünschten Bestandteile in ein anderes Gefäß überführt, was zu Kontaminationen führen kann. Vielmehr wird diejenige überstehende Flüssigkeit abgetrennt, die anschließend verworfen wird, während die gewünschten Bestandteile adsorbiert werden und weiterhin im Gefäß verbleiben. Somit ist bei dem vorliegenden Verfahren die Gefahr einer Kontamination minimiert.

Vorteilhafterweise werden für die Zentrifugation Geschwindigkeiten von 100 bis 500.000 UpM, weiter bevorzugt von 500 bis 13.000 UpM, am meisten bevorzugt von 1.000 bis 5.000 UpM angewandt.

Es hast sich ebenfalls gezeigt, dass bei einer Reduktion der Probenmenge auf den Waschschritt verzichtet werden kann, ohne Ausbeuteverluste zu erleiden. Bei den üblich eingesetzten Probenmengen von etwa 20 l befinden sich im Sediment der adsorbierten bzw. adhäsierten Zellorganellen derartige Mengen an Inhibitoren, dass nachfolgende Anwendungen wie PCR-Amplifikation beeinträchtigt werden können. Durch Waschschritte kann eine Störung durch Inhibitoren verringert oder ganz verhindert und die Ausbeute erhöht werden. Eine Reduktion der Probenmenge führt jedoch ebenfalls dazu, dass die Menge an Inhibitoren verringert wird, auch ohne dass Waschschritte stattfinden. Gleichzeitig ist die Bindung der Nukleinsäure an die Matrix, insbesondere bei zusätzlicher Anwendung eines oder mehrerer Zentrifugationsschritte, anscheinend derart stark, dass es nicht zu Ausbeutverlusten kommt.

Ein weiterer Gegenstand der vorliegenden Erfindung ist ein Kit zur Gewinnung von Nukleinsäure aus Proben enthaltend Zellorganellen, die Nukleinsäuren enthalten, umfassend
a) ein Matrixmaterial, das sich entweder in festem Zustand an der inneren Wand eines oder mehrerer leerer Reaktionsgefäße befindet oder als Feststoff, in Lösung oder in dispergiertem Zustand vorliegt, und das in der Lage ist, Zellorganellen zu adsorbieren,
b) ein oder mehrere Erythrozyten-Lysepuffer,
c) wenn sich das Matrixmaterial nicht an der inneren Wand der ein oder mehreren leeren Reaktionsgefäße befindet, optional ein oder mehrere Reaktionsgefäße,
d) optional ein oder mehrere Waschlösungen,
e) optional Reagenzien zur Durchführung von PCR-Reaktionen und
f) optional eine Gebrauchsanleitung.

Als Matrixmaterial findet vorzugsweise das oben beschriebene, zur Ausbildung einer polyanionischen Struktur befähigte Polymer, Copolymer oder Terpolymer Anwendung.

Bei den Reaktionsgefäßen handelt es sich vorzugsweise um Probengefäße wie sie im Stand der Technik zur Aufreinigung biologischer Proben verwendet werden. Dabei kann es sich um Einzelgefäße oder um ein Konglomerat mehrerer Gefäße handeln, wie beispielsweise um 48- oder 96-Well-Platten oder um andere aus dem Stand der Technik bekannte Gefäßkonglomerate.

Das Matrixmaterial befindet sich vorzugsweise an der inneren Wand dieser Gefäße. Es kann dabei entweder wie oben beschrieben vorab an der Wand fixiert oder adsorbiert sein, so dass ein oder mehrere bereits beschichtete Probengefäße zur Verfügung gestellt werden. Es besteht auch die Möglichkeit, dass das Matrixmaterial als Feststoff, in Lösung oder in dispergiertem Zustand zur Verfügung gestellt wird und im Verlauf des oben beschriebenen Verfahrens zur Gewinnung der Nukleinsäure zugegeben wird. Es kann dabei sowohl zu der Lyselösung als auch zu der Probe zugegeben werden, es kann in dem leeren Reaktiönsgefäß vorgelegt werden oder man kann es dem Lysat zufügen.

Das Matrixmaterial und das Material der Probengefäße sollten so gewählt werden, dass das Matrixmaterial nach der Lyse an den inneren Wänden des Reaktionsgefäßes fixiert oder adsorbiert ist. Bei Verwendung der oben beschriebenen Polymere können handelsübliche Probengefäße, insbesondere aus Plastik, verwendet werden. Die Verwendung von PCR-Reaktionsgefäßen hat den Vorteil, dass die vorbereiteten Proben zur Durchführung der PCR-Reaktion nicht mehr umgefüllt werden müssen.

Insbesondere wenn das Matrixmaterial noch nicht als Beschichtung der Probengefäße vorliegt, können dem Kit auch ein oder mehrere der Probengefäße beigefügt werden.

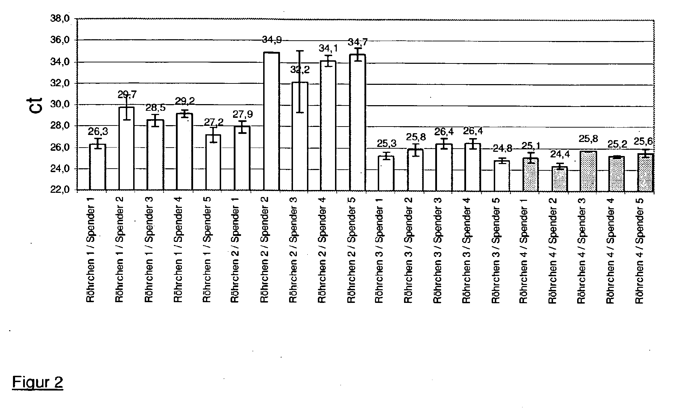

Anhand der nachfolgenden Beispiele soll die vorliegende Erfindung erläutert werden. Die Beispiele verkörpern lediglich vorteilhafte Ausführungsformen der Erfindung, ohne sie zu beschränken.

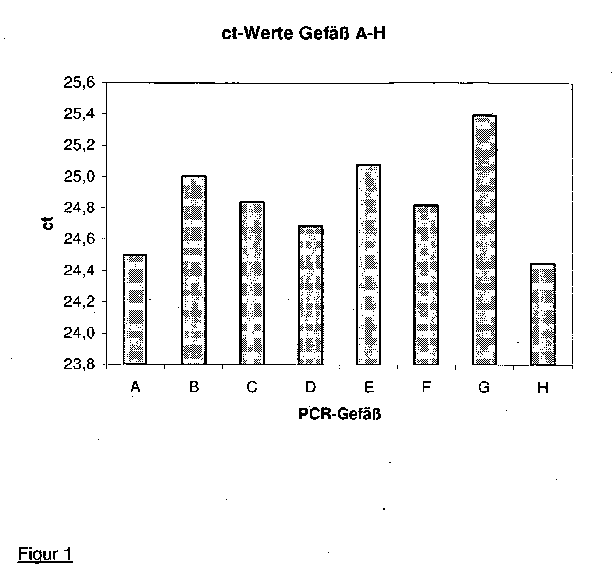

Fig. 1 ist folgendes zu entnehmen:
   8 Proben wurden in PCR-Gefäßen gemäß Beispiel 10, die gemäß Beispiel 9 , mit einer Poly(vinylmethylether-maleinsäure)-Beschichtung versehen waren, amplifiziert. Die erhaltenen Ergebnisse sind in Form der ct-Werte in Fig. 1 wiedergegeben.
Fig. 2 ist folgendes zu entnehmen:
   Blut von 5 Spendern wurde in jeweils 4 verschiedenen Blutentnahmeröhrchen gesammelt. Für jede Probe/Blutentnahmeröhrchenkombination wurden 4 Präparationen in PCR-Gefäßen - wie in Beispiel 10 beschrieben - durchgeführt. Bei der qPCR wurde ein Sequenzabschnitt des humanen ß-Aktin Gens mit Hilfe von sog. TaqMan-Probes amplifiziert. Das Balkendiagramm der Fig. 2 stellt die Ergebnisse der quantitativen PCR dar. Jeder Balken gibt den über vier PCR-Gefäßen gemittelten ct-Wert wieder.

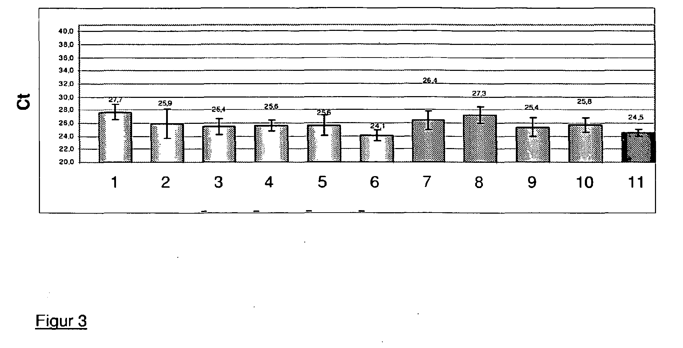

Fig. 3 zeigt die ct-Werte der PCR-Reaktionen von vorbereiteten Proben entsprechend Beispiel 11 in Probengefäßen, die mit unterschiedlichen Polymeren beschichtet wurden.

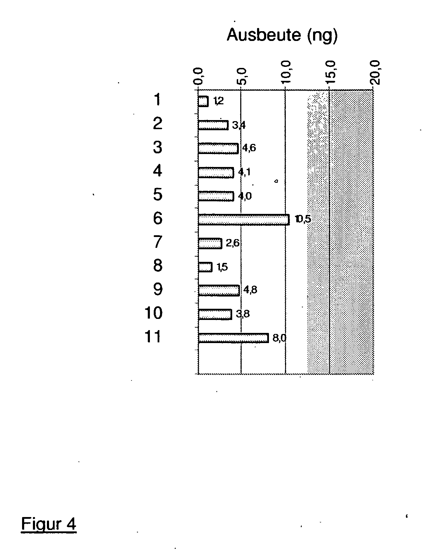

Fig. 4 zeigt die unter Verwendung einer Eichkurve erhaltenen berechneten absoluten Mengen an Nukleinsäure, die bei der Amplifikation entsprechend Beispiel 11 resultieren.

### Beispiele

1.) Zu 50 ml Polystyrol-co-maleinsäure mit einem 14%-igen Gewichtsanteil an Maleinsäureanhydrid gelöst in DMSO (20 mg/ml Dimethylsulfoxid) gibt man 250 mg N-(2-Hydroxyethyl-iminodiessigsäure) und erhitzt über einen Zeitraum von 45 min auf 60 °C.
   Anschließend verdünnt man mit DMSO 10-fach und erhält somit eine gebrauchsfertige Lösung zur Beschichtung von Reaktionsgefäßen für die Amplifikationsreaktionen bzw. PCR. 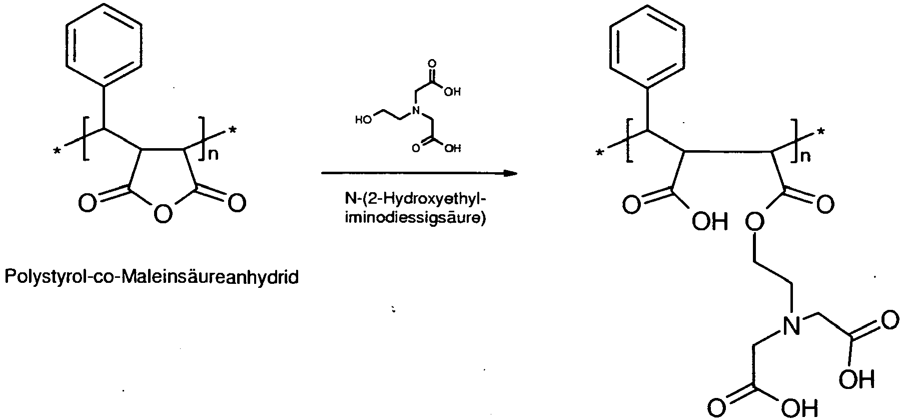
2.) Zu 50ml Polystyrol-co-maleinsäure mit einem 50%-igen Gewichtsanteil an Maleinsäureanhydrid gelöst in DMSO (20mg/ml Dimethylsulfoxid) gibt man 1,3 g N-(2-Hydroxyethyl)-iminobis(methylphosphonsäure) und erhitzt über einen Zeitraum 1 h auf eine Temperatur von 60 °C. Anschließend verdünnt man mit DMSO 10-fach und erhält somit eine gebrauchsfertige Lösung zur Beschichtung von Reaktionsgefäßen für Amplifikationsreaktionen bzw. PCR. 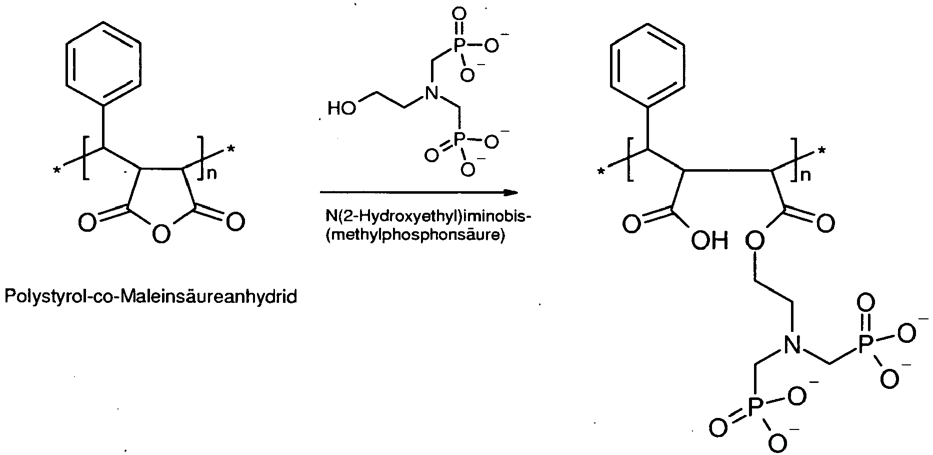
3.) 50 mg (0.877 mmol) N-Me-PVA (N-Methyl-polyvinylamin) wurden in 2 ml Wasser gelöst und nach Zugabe von 1.31 mmol Bromessigsäure (180 mg, 1,5 eq) um 50 µl TEA (Triethylamin) über einen Zeitraum von 12 h geschüttelt. Man erhält so eine gebrauchsfertige Lösung zur Beschichtung von Reaktionsgefäßen für die Amplifikationsreaktionen bzw. PCR.
4.) 0.2 g Polyvinylalkohol (MW 9000 -10000) wurden mit 630 mg (0,454 mmol, 0,8 eq.) Bromessigsäure versetzt und nach Zugabe von 1,2 g K₂CO₃ (9,08 mmol, 1,5 eq.) wurde für 12 h geschüttelt. Es resultiert eine gebrauchsfertige Lösung zur Beschichtung von Reaktionsgefäßen für Amplifikationsreaktionen bzw. PCR.
5)
   a) Synthese von Methacrylsäure-N-(4-methylphenol)amid 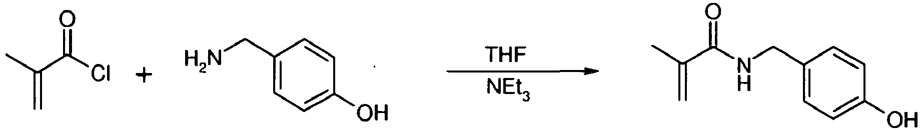 302 mg (2.2 mmol) Tyramin werden in 5 mL THF gelöst, mit 570 µL Triethylamin versetzt und auf 0°C gekühlt. Nach langsamer Zugabe von 208 mg (2 mmol) Methacrylsäurechlorid wird über Nacht gerührt. Anschließend wird mit 1 M Salzsäure hydrolysiert und 3x mit Ethylacetat extrahiert. Die gesammelten organischen Phasen werden über Natriumsulfat getrocknet und anschließend wird das Lösungsmittel unter vermindertem Druck entfernt. (1.52 mmol, Ausbeute 76%)
   b) Synthese von Poly(methylacrylsäure-N-(4-methylphenol)amid) 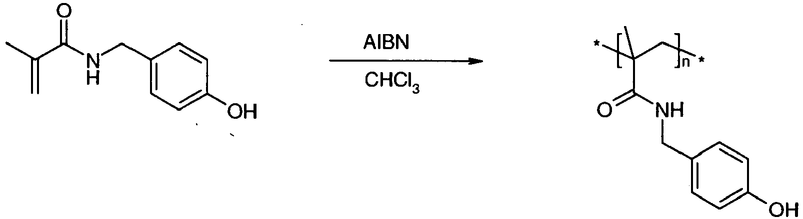
   Das aus a) erhaltene Monomer wurde in Chloroform gelöst und nach Zugabe einer Spatelspitze AIBN wurde für 5h unter Rückfluss gekocht. Dann wurde nochmals eine Spatelspitze AIBN zugegeben und über Nacht gekocht. Anschließend wurde hydrolysiert und 2x mit Dichlormethan extrahiert. Die gesammelten organischen Phasen wurden über Natriumsulfat getrocknet und anschließend wurde das Lösungsmittel unter vermindertem Druck entfernt. Es wurde ein rötliches Harz erhalten, das nicht weiter aufgereinigt wurde.
   Es wurde eine Beschichtungslösung mit einer Konzentration von 60mg/ml in Ethanol hergestellt.
6)
   a) Synthese von Methacrylsäure-2-hydroxy-3-aminopropyl-N-(4-methylphenol)ester 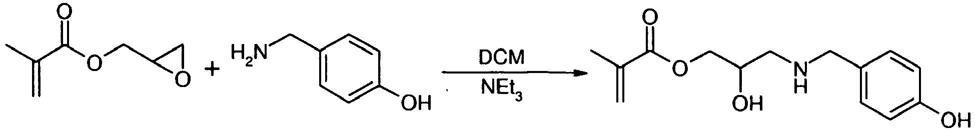 302 mg (2.2 mmol) Tyramin und 284 mg (2 mmol) Methacrylsäure-Glycidolester wurden in 5 mL Dichlormethan (DCM) vorgelegt und nach langsamer Zugabe von 300 µL Triethylamin über Nacht bei RT gerührt. Anschließend wurde mit 1 M Salzsäure hydrolysiert und 3x mit Ethylacetat extrahiert. Die gesammelten organischen Phasen wurden über Natriumsulfat getrocknet und anschließend wurde das Lösungsmittel unter vermindertem Druck entfernt. (1,36 mol, Ausbeute 68%)
   b) Synthese von Poly(methacrylsäure-2-hydroxy-3-aminopropyl-N-(4-methylphenol)ester) 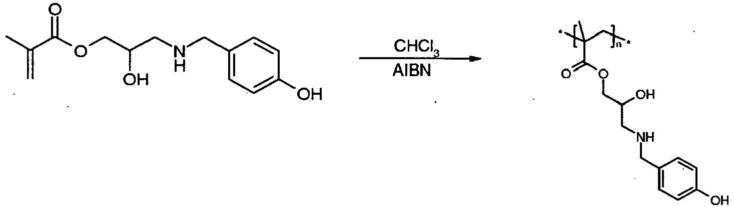 Das Monomer wurde in Chloroform gelöst und nach Zugabe einer Spatelspitze AIBN wurde für 5h unter Rückfluss gekocht. Dann wurde nochmals eine Spatelspitze AIBN zugegeben und über Nacht gekocht. Anschließend wurde hydrolysiert und 2x mit Dichlormethan extrahiert. Die gesammelten organischen Phasen wurden über Natriumsulfat getrocknet und anschließend wurde das Lösungsmittel unter vermindertem Druck entfernt. Es wurde ein hellgelbes Harz erhalten, das mittels Dialyse aufgereinigt wurde.
      Zur Herstellung einer Beschichtungslösung (analog zu Beispiel 9) wurde als Lösungsmittel Wasser verwendet.
7)
   Synthese von Poly(maleinsäure-N-(4-methylphenol)amid) 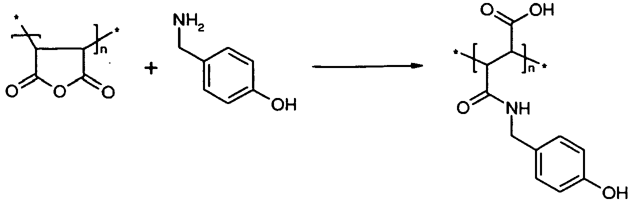 500 mg Poly(maleinsäureanhydrid) (~5,1 mmol Maleinsäureanhydrid) wurden mit 680 mg (4,95 mmol) Tyramin vermengt und für 12h bei 60 °C gerührt. Anschließend wurde zum Reinigen dialysiert.
   Es wurde eine Beschichtungslösung mit einer Konzentration von 60mg/ml in VE-Wasser hergestellt.
8) Poly(weinsäuresuccinat) 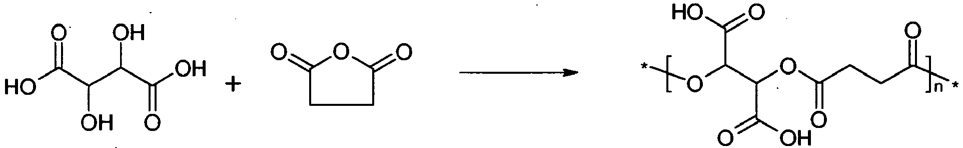 1,5 g (10 mmol) Weinsäure wurden mit 1g (10 mmol) Bernsteinsäureanhydrid gemischt und für 5h auf 60°C erhitzt. Es entstand ein blassgelbes Harz, welches durch Dialyse aufgereinigt wurde.
   Es wurde eine Beschichtungslösung mit einer Konzentration von 60mg/ml in VE-Wasser hergestellt.
9) Durchführung der Beschichtung
   20 mg Poly(methylvinylether-alt-maleinsäure) wurden in 10 ml VE-Wasser gelöst. Nachdem das Polymer vollständig gelöst war, wurden in handelsübliche PCR-Gefäße (8-ter Strips) aus Polypropylen jeweils 50 µl der wässrigen Polymerlösung pipettiert. Nach 20 min Inkubationszeit bei Raumtemperatur wurde die wässrige Polymerlösung aus den PCR-Gefäßen pipettiert. Abschließend wurden die Gefäße einmal mit 120 µl Wasser gespült und bei 40°C getrocknet.
10) Präparation der Blutprobe im PCR-Gefäß:
   In den Kavitäten von PCR-Gefäßen wurden je 50µl Lysepuffer (155 mM NH₄Cl, 10 mM KHCO₃ in Wasser) vorgelegt und 10 µl Blut hinzupipettiert. Durch 10-maliges auf- und abpipettieren wurde die Blutprobe mit dem Lysepuffer vollständig vermischt. Nach einer Inkubationszeit von 5 min wurde erneut vermischt und nach weiteren 15 min wurde der flüssige Inhalt der PCR-Gefäße verworfen, dann einmal mit 120 µl Waschpuffer (1 mM Tris-HCl, 0,5mM EDTA, pH 7,4 + 0,05% Nonidet P-40) gewaschen und rückstandslos verworfen. Anschließend wurden 25 µl eines PCR Reaktionsgemisches zugegeben und eine quantitative "real time" PCR (RT-PCR) durchgeführt. Die entsprechenden Resultate sind in Fig.2 wiedergegeben.
11)
   In einem weiteren Versuch wurden auf einer 96-Well-Platte jeweils 2 Reihen à 8 Wells mit folgenden Polymeren beschichtet:
   1+2: Poly(methylacrylsäure-N- (4-methylphenol)amid) aus Beispiel 5
   3+4: Poly(maleinsäure-N-propylamid)
   5+6: Poly(maleinsäure-N- (4-methylphenol)amid) aus Beispiel 7
   7+8: Poly(weinsäuresuccinat) aus Beispiel 8
   9+10: Poly(isobutylen-alt-maleinsäure)
   11: Poly(methylvinylether-alt-maleinsäure) aus Beispiel 9

Zur Beschichtung wurden die in den jeweiligen aufgeführten Beispielen beschriebenen Beschichtungslösungen hergestellt. Die Polymaleinsäure-N-propylamid-Lösung wurde in einer Konzentration von 40mg/ml in VE-Wasser hergestellt. Die Poly(isobutylen-alt-maleinsäure) wurde in einer Konzentration von 5 l/ml in 1 N KOH hergestellt.

Nachdem das Polymer vollständig gelöst war, wurden in handelsübliche PCR-Gefäße (8-ter Strips) aus Polypropylen jeweils 50 µl der Polymerlösung pipettiert. Nach 5 min Inkubationszeit bei Raumtemperatur wurde der flüssige Inhalt der PCR-Gefäße abpipettiert. Anschließend wurden die Gefäße 1 Stunde bei 50°C getrocknet.

Anschließend wurde eine Präparation einer Blutprobe sowie anschließende PCR-Reaktion gemäß der Beschreibung aus Beispiel 10 durchgeführt. Die bei den verwendeten Polymeren erhaltenen Ct-Werte sind in Figur 3 dargestellt, die unter Verwendung einer Eichkurve erhaltenen, berechneten absoluten Ausbeuten zeigt Figur 4.

Die in den beiden Figuren dargestellten Ergebnisse zeigen, dass auch Polyphenole und Polyester als Beschichtungsmaterialien gemäß der vorliegenden Erfindung geeignet sind.

## Patentansprüche

1. Verfahren zur Gewinnung von Nukleinsäuren aus Blut, **gekennzeichnet durch** die folgenden Verfahrensschritte:
a) In Kontakt bringen einer Blutprobe in einem Reaktionsgefäß mit einem Erythrozyten-Lysepuffer in Gegenwart einer Matrix, welche die Zellorganellen adsorbieren kann;
b) Entfernen der aus der Lyse resultierenden Reaktionsmischung;
c) ggf. Waschen der Matrix;
d) Erwärmen der Probe auf eine Temperatur, bei der die Nukleinsäuren aus den anhaftenden Blutbestandteilen freigesetzt werden;
e) ggf. Unterwerfen der Probe einer Folgereaktion,
wobei das Matrixmaterial im festen Zustand an der Wand des Reaktionsgefäßes adsorbiert ist,
oder wobei das Matrixmaterial am Anfang der Lyse in einem flüssigen oder dispersen Zustand vorliegt und nach der Lyse an den Wänden des Reaktionsgefäßes adsorbiert ist,
wobei das Matrixmaterial ein zur Ausbildung einer polyanionischen Struktur befähigtes Polymer, Copolymer oder. Terpolymer oder Mischungen derselben ist, und
wobei das Reaktionsgefäß aus Plastik besteht.

2. Verfahren nach Anspruch 1, **dadurch gekennzeichnet, dass** die Nukleinsäure genomische DNA verkörpert.

3. Verfahren nach Anspruch 1 oder 2 **dadurch gekennzeichnet, dass** das zur Ausbildung einer polyanionischen Struktur befähigte Polymer, Copolymer oder Terpolymer ein Polycarboxylat oder ein carboxyliertes Polymer oder ein zur Ausbildung einer polyanionischen Struktur befähigter Polyester ist.

4. Verfahren nach Anspruch 3, **dadurch gekennzeichnet, dass** das zur Ausbildung einer polyanionischen Struktur befähigte Polymer, Co- oder Terpolymer ein carboxyliertes Polymer auf der Basis von Vinylmethylether, Maleinsäureanhydrid, Styrol, unverzweigten oder verzweigten Alkenen oder Acrylsäure und deren Derivaten ist.

5. Verfahren nach Anspruch 4, **dadurch gekennzeichnet, dass** das Acrylsäurederivat ein verzweigter oder unverzweigter C₁-C₁₂-Alkylester der Acryl- oder Methacrylsäure mit einem bis zwölf Kohlenstoffatomen, vorzugsweise Methylmethacrylat, Ethylmethacrylat, Butylmethacrylat, 2-Ethylhexylmethacrylat, Methylacrylat, Ethylacrylat, Butylacrylat und 2-Ethylhexyacrylat ist.

6. Verfahren nach Anspruch 3, **dadurch gekennzeichnet, dass** das zur Ausbildung einer polyanionischen Struktur befähigte Polymer, Copolymer oder Terpolymer Phosphonsäuregruppen oder Sulfonsäuregruppen oder Phenolgruppen, vorzugsweise Tyramingruppen, oder Mischungen derselben aufweist.

7. Verfahren nach Anspruch 6, **dadurch gekennzeichnet, dass** das Sulfonsäuregruppen aufweisende Polymer Polystyrolsulfonsäure oder ein Copolymer/Terpolymer mit Polystyrolsulfonsäure ist.

8. Verfahren nach Anspruch 3, **dadurch gekennzeichnet, dass** das zur Ausbildung einer polyanionischen Struktur befähigte Co- oder Terpolymer ein carboxyliertes Polymer auf der Basis von Styrol und Maleinsäureanhydrid ist.

9. Verfahren nach Anspruch 8, **dadurch gekennzeichnet, dass** das Copolymere 5 bis 95 Gewichtsprozent, vorzugsweise 25 bis 95 Gewichtsprozent, weiter bevorzugt 50 bis 95 Gewichtsprozent Maleinsäureeinheiten enthält.

10. Verfahren nach Anspruch 3, **dadurch gekennzeichnet, dass** das zur Ausbildung einer polyanionischen Struktur befähigte Co- oder Terpolymer ein carboxyliertes Polymer auf der Basis von Methylvinylether und Maleinsäure, vorzugsweise das Copolymer Poly(methylvinylether-alt-maleinsäure) 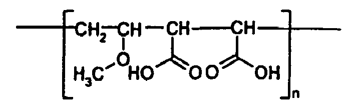 ist, wobei das Poly(methylvinylether-alt-maleinsäure)-Copolymer bevorzugt ein Molekulargewicht in einem Bereich von 1.0·10³ bis 2.5·10⁶, vorzugsweise in einem Bereich von 1.0·10⁴ bis 2.2·10⁶, weiter bevorzugt in einem Bereich von 1.9·10⁶ bis 2.1·10⁶ aufweist.

11. Verfahren nach Anspruch 10, **dadurch gekennzeichnet, dass** das Copolymer Poly(methylvinylether-alt-maleinsäure) als partieller Methylester vorliegt.

12. Verfahren nach Anspruch 1 oder 2, **dadurch gekennzeichnet, dass** zur Erythrozytenlyse ein Puffer eingesetzt wird, der ein oder mehrere Salze eines Alkali-, Pseudoalkali- oder Erdalkalimetallsalzes mit anorganischen oder organischen Säuren, ein oder ggf. mehrere kationische Detergenzien sowie ggf. eine den pH-Wert puffernde Substanz enthält.

13. Verfahren nach Anspruch 1 oder 2, **dadurch gekennzeichnet, dass** als Waschpuffer Wasser oder ein Puffer, enthaltend TRIS, Komplexbildner und ggf. ein Detergens eingesetzt werden.

14. Verfahren nach Anspruch 1 oder 2, **dadurch gekennzeichnet, dass** die an der Matrix adsorbierten Blutbestandteile in Verfahrensschritt d) auf eine Temperatur in einem Intervall von 45 bis 100 °C, vorzugsweise von 80 bis 98 °C, weiter bevorzugt von 94-96 °C, erhitzt werden.
wobei das Erhitzen nach dem optionalen Waschen in Verfahrensschritt c) erfolgt,
wobei das Erhitzen ggf. in wässriger Lösung stattfindet und
wobei die in Schritt d) freigesetzten Nukleinsäuren ggf. in Verfahrensschritt e) einer Amplifikationsreaktion oder einer PCR unterworfen werden.

15. Verfahren nach einem der Ansprüche 1 bis 14, **dadurch gekennzeichnet, dass** nach dem in Kontakt Bringen der Blutprobe mit dem Erythrozyten-Lysepuffer und der Matrix und vor dem Entfernen der aus der Lyse resultierenden Reaktionsmischung ein oder mehrere Zentrifugationsschritte stattfinden.

16. Kit zur Gewinnung von Nukleinsäure aus Blut enthaltend
a) ein Matrixmaterial, das entweder in festem Zustand an der inneren Wandung eines oder mehrerer leerer Reaktionsgefäße adsorbiert ist oder das am Anfang der Lyse als Feststoff, in Lösung oder in dispergiertem Zustand vorliegt und das nach der Lyse an den Wänden des Reaktionsgefäßes adsorbiert ist, und das in der Lage ist, Zellorganellen zu adsorbieren,
b) ein oder mehrere Erythrozyten-Lysepuffer,
c) wenn sich das Matrixmaterial nicht an der inneren Wand der ein oder mehreren leeren Reaktionsgefäße befindet, optional ein oder mehrere Reaktionsgefäße,
d) optional ein oder mehrere Waschlösungen,
e) optional Reagenzien zur Durchführung von PCR-Reaktionen und
f) optional eine Gebrauchsanleitung,
wobei es sich bei dem Matrixmaterial um das in den Ansprüchen 1 und 3 bis 11 beschriebene Material handelt,
wobei das Matrixmaterial ein zur Ausbildung einer polyanionischen Struktur befähigtes Polymer, Copolymer oder Terpolymer oder Mischungen derselben ist.

## Claims

1. Method for extracting nucleic acids from blood, **characterized by** the following method steps:
a) bringing a blood sample in a reaction vessel into contact with an erythrocyte lysis buffer in the presence of a matrix which can adsorb the cell organelles;
b) removing the reaction mixture resulting from the lysis;
c) optionally washing the matrix;
d) heating the sample to a temperature at which the nucleic acids are released from the adhering blood constituents;
e) optionally subjecting the sample to a subsequent reaction,
wherein the matrix material is adsorbed in the solid state on the wall of the reaction vessel,
or wherein the matrix material is present in a liquid or disperse state at the start of the lysis and is adsorbed on the walls of the reaction vessel after the lysis,
wherein the matrix material is a polymer, copolymer or terpolymer capable of forming a polyanionic structure, or mixtures of the same,
and wherein the reaction vessel consists of plastic.

2. Method according to Claim 1, **characterized in that** the nucleic acid embodies genomic DNA.

3. Method according to Claim 1 or 2, **characterized in that** the polymer, copolymer or terpolymer capable of forming a polyanionic structure is a polycarboxylate or a carboxylated polymer or a polyester capable of forming a polyanionic structure.

4. Method according to Claim 3, **characterized in that** the polymer, copolymer or terpolymer capable of forming a polyanionic structure is a carboxylated polymer based on vinyl methyl ether, maleic anhydride, styrene, unbranched or branched alkenes or acrylic acid and derivatives thereof.

5. Method according to Claim 4, **characterized in that** the acrylic acid derivative is a branched or unbranched C₁-C₁₂-alkyl ester of acrylic or methacrylic acid having one to twelve carbon atoms, preferably methyl methacrylate, ethyl methacrylate, butyl methacrylate, 2-ethylhexyl methacrylate, methyl acrylate, ethyl acrylate, butyl acrylate and 2-ethylhexyl acrylate.

6. Method according to Claim 3, **characterized in that** the polymer, copolymer or terpolymer capable of forming a polyanionic structure has phosphonic acid groups or sulphonic acid groups or phenol groups, preferably tyramine groups, or mixtures of the same.

7. Method according to Claim 6, **characterized in that** the polymer having sulphonic acid groups is polystyrene sulphonic acid or a copolymer/terpolymer with polystyrene sulphonic acid.

8. Method according to Claim 3, **characterized in that** the copolymer or terpolymer capable of forming a polyanionic structure is a carboxylated polymer based on styrene and maleic anhydride.

9. Method according to Claim 8, **characterized in that** the copolymer comprises 5 to 95 per cent by weight, preferably 25 to 95 per cent by weight, more preferably 50 to 95 per cent by weight of maleic acid units.

10. Method according to Claim 3, **characterized in that** the copolymer or terpolymer capable of forming a polyanionic structure is a carboxylated polymer based on methyl vinyl ether and maleic acid, preferably the copolymer poly(methyl vinyl ether-alt- maleic acid) 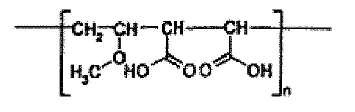 wherein the poly(methyl vinyl ether-alt- maleic acid) copolymer preferably has a molecular weight in a range from 1.0˙10³ to 2.5˙10⁶, preferably in a range from 1.0·10⁴ to 2.2·10⁶, more preferably in a range from 1.9·10⁶ to 2.1·10⁶.

11. Method according to Claim 10, **characterized in that** the copolymer poly(methyl vinyl ether-*alt-*maleic acid) is present as the partial methyl ester.

12. Method according to Claim 1 or 2, **characterized in that** a buffer is used for erythrocyte lysis which comprises one or more salts of an alkali metal, pseuodoalkali metal or alkaline earth metal with inorganic or organic acids, one or optionally more cationic detergents and optionally a substance for buffering the pH.

13. Method according to Claim 1 or 2, **characterized in that** water or a buffer comprising TRIS, complexing agent and optionally a detergent are used as washing buffer.

14. Method according to Claim 1 or 2, **characterized in that** the blood constituents adsorbed on the matrix in method step d) are heated to a temperature in an interval from 45 to 100°C, preferably 80 to 98°C, more preferably 94-96°C, wherein the heating takes place after the optional washing in method step c),
wherein the heating is conducted optionally in aqueous solution and
wherein the nucleic acids released in step d) are optionally subjected to an amplification reaction or a PCR in method step e).

15. Method according to any of Claims 1 to 14, **characterized in that** one or more centrifugation steps are carried out after bringing the blood sample into contact with the erythrocyte lysis buffer and the matrix and prior to removing the reaction mixture resulting from the lysis.

16. Kit for extracting nucleic acid from blood comprising
a) a matrix material, which is either adsorbed in the solid state on the inner wall of one or more empty reaction vessels or is present as a solid, in solution or in dispersed form at the start of the lysis and is adsorbed on the walls of the reaction vessel after the lysis, and which is able to adsorb cell organelles,
b) one or more erythrocyte lysis buffers,
c) optionally one or more reaction vessels if the matrix material is not present on the inner wall of the one or more empty reaction vessels,
d) optionally one or more washing solutions,
e) optionally reagents for carrying out PCR reactions and
f) optionally an instruction manual,
wherein the matrix material is the material described in Claims 1 and 3 to 11,
wherein the matrix material is a polymer, copolymer or terpolymer capable of forming a polyanionic structure, or mixtures of the same.

## Revendications

1. Procédé pour l'extraction d'acides nucléiques à partir de sang, **caractérisé par** les étapes de processus suivantes :
a) mise en contact d'un échantillon de sang dans un récipient de réaction avec un tampon de lyse d'érythrocytes en présence d'une matrice qui peut adsorber les organites cellulaires ;
b) élimination du mélange réactionnel résultant de la lyse ;
c) éventuellement lavage de la matrice ;
d) chauffage de l'échantillon jusqu'à une température à laquelle les acides nucléiques sont libérés des composants adhérents du sang ;
e) éventuellement soumission de l'échantillon à une réaction subséquente,
le matériau de la matrice étant adsorbé à l'état solide sur la paroi du récipient de réaction,
ou le matériau de la matrice se trouvant au début de la lyse en un état liquide ou dispersé et étant après la lyse adsorbé sur les parois du récipient de réaction,
le matériau de la matrice consistant en un polymère, copolymère ou terpolymère apte à la formation d'une structure polyanionique ou en des mélanges de ceux-ci, et le récipient de réaction étant constitué de matière plastique.

2. Procédé selon la revendication 1, **caractérisé en ce que** l'acide nucléique représente un ADN génomique.

3. Procédé selon la revendication 1 ou 2, **caractérisé en ce que** le polymère, copolymère ou terpolymère apte à la formation d'une structure polyanionique est un polycarboxylate ou un polymère carboxylé ou un polyester apte à la formation d'une structure polyanionique.

4. Procédé selon la revendication 3, **caractérisé en ce que** le polymère, copolymère ou terpolymère apte à la formation d'une structure polyanionique est un polymère carboxylé à base d'éther vinylméthylique, d'anhydride maléique, de styrène, d'alcènes ramifiés ou non ramifiés ou d'acide acrylique et de ses dérivés.

5. Procédé selon la revendication 4, **caractérisé en ce que** le dérivé d'acide acrylique est un ester alkylique en C₁-C₁₂ ramifié ou non ramifié de l'acide acrylique ou méthacrylique ayant de un à douze atomes de carbone, de préférence le méthacrylate de méthyle, le méthacrylate d'éthyle, le méthacrylate de butyle, le méthacrylate de 2-éthylhexyle, l'acrylate de méthyle, l'acrylate d'éthyle, l'acrylate de butyle et l'acrylate de 2-éthylhexyle.

6. Procédé selon la revendication 3, **caractérisé en ce que** le polymère, copolymère ou terpolymère apte à la formation d'une structure polyanionique comporte des groupes phosphono ou des groupes sulfo ou des groupes phénol, de préférence des groupes tyramino, ou des mélanges de ceux-ci.

7. Procédé selon la revendication 6, **caractérisé en ce que** le polymère comportant des groupes sulfo est le poly(acide styrènesulfonique) ou un copolymère/ terpolymère avec le poly(acide styrènesulfonique).

8. Procédé selon la revendication 3, **caractérisé en ce que** le copolymère ou terpolymère apte à la formation d'une structure polyanionique est un polymère carboxylé à base de styrène et d'anhydride maléique.

9. Procédé selon la revendication 8, **caractérisé en ce que** le copolymère contient 5 à 95 pour cent en poids, de préférence 25 à 95 pour cent en poids, encore mieux 50 à 95 pour cent en poids de motifs acide maléique.

10. Procédé selon la revendication 3, **caractérisé en ce que** le copolymère ou terpolymère apte à la formation d'une structure polyanionique est un polymère carboxylé à base d'éther méthylvinylique et d'acide maléique, de préférence le copolymère poly(éther méthylvinylique-alt-acide maléique) 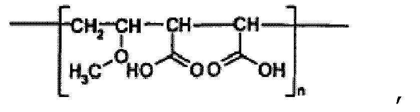 le copolymère poly(éther méthylvinylique-alt-acide maléique) présentant de préférence une masse moléculaire dans une plage de 1,0.10³ à 2,5.10⁶, de préférence dans une plage de 1,0.10⁴ à 2,2.10⁶, encore mieux dans une plage de 1,9.10⁶ à 2,1.10⁶.

11. Procédé selon la revendication 10, **caractérisé en ce que** le copolymère poly(éther méthylvinylique-alt-acide maléique) se trouve sous forme d'ester méthylique partiel.

12. Procédé selon la revendication 1 ou 2, **caractérisé en ce qu'**on utilise pour la lyse des érythrocytes un tampon qui contient un ou plusieurs sels d'un métal alcalin, pseudo-alcalin, ou alcalino-terreux avec des acides organiques ou inorganiques, un ou éventuellement plusieurs détergents cationiques ainsi qu'éventuellement une substance tamponnant le pH.

13. Procédé selon la revendication 1 ou 2, **caractérisé en ce qu'**on utilise comme tampon de lavage l'eau ou un tampon contenant du TRIS, un complexant et éventuellement un détergent.

14. Procédé selon la revendication 1 ou 2, **caractérisé en ce que** dans l'étape d) du procédé on chauffe les composants du sang adsorbés sur la matrice jusqu'à une température dans une plage de 45 à 100 °C, de préférence de 80 à 98 °C, encore mieux de 94-96 °C, le chauffage s'effectuant après le lavage optionnel dans l'étape c) du procédé,
le chauffage ayant éventuellement lieu en solution aqueuse et
les acides nucléiques libérés dans l'étape d) étant éventuellement soumis dans l'étape e) du procédé à une réaction d'amplification ou à une PCR.

15. Procédé selon l'une quelconque des revendications 1 à 14, **caractérisé en ce qu'**après la mise en contact de l'échantillon de sang avec le tampon de lyse d'érythrocytes et la matrice et avant l'élimination du mélange réactionnel résultant de la lyse une ou plusieurs étapes de centrifugation ont lieu.

16. Nécessaire pour l'extraction d'acide nucléique à partir de sang, contenant
a) un matériau de matrice, soit qui est adsorbé à l'état solide sur la paroi interne d'un ou de plusieurs récipients de réaction vides, soit qui se trouve au début de la lyse sous forme de solide, en solution ou à l'état dispersé et qui après la lyse est adsorbé sur les parois du récipient de réaction, et qui est apte à adsorber des organites cellulaires,
b) un ou plusieurs tampons de lyse d'érythrocytes,
c) lorsque le matériau de la matrice ne se trouve pas sur la paroi interne dudit/desdits un ou plusieurs récipients de réaction vides, en option un ou plusieurs récipients de réaction,
d) en option une ou plusieurs solutions de lavage,
e) en option des réactifs pour l'exécution de réactions PCR et
f) en option un mode d'emploi,
le matériau de la matrice consistant en le matériau décrit dans les revendications 1 et 3 à 11,
le matériau de la matrice consistant en un polymère, copolymère ou terpolymère apte à la formation d'une structure polyanionique ou en des mélanges de ceux-ci.
